(19) Europäisches Patentamt
European Patent Office
Office européen des brevets

(11) **EP 3 813 673 B1**

(12) **EUROPEAN PATENT SPECIFICATION**

(45) Date of publication and mention
of the grant of the patent:
**14.12.2022 Bulletin 2022/50**

(21) Application number: **19733050.9**

(22) Date of filing: **28.06.2019**

(51) International Patent Classification (IPC):
*A61B 8/06* *(2006.01)*      *A61B 8/08* *(2006.01)*
*A61B 8/00* *(2006.01)*

(52) Cooperative Patent Classification (CPC):
**A61B 8/488; A61B 8/06; A61B 8/065;**
**A61B 8/0883; A61B 8/469; A61B 8/483;**
**A61B 8/5223; G16H 50/30;** A61B 8/5292

(86) International application number:
**PCT/EP2019/067288**

(87) International publication number:
**WO 2020/002572 (02.01.2020 Gazette 2020/01)**

(54) **METHODS AND SYSTEMS FOR PERFORMING TRANSVALVULAR PRESSURE QUANTIFICATION**

VERFAHREN UND SYSTEME ZUR DURCHFÜHRUNG DER QUANTIFIZIERUNG VON TRANSVALVULAREM DRUCK

PROCÉDÉS ET SYSTÈMES PERMETTANT D'EFFECTUER UNE QUANTIFICATION DE PRESSION TRANSVALVULAIRE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.06.2018 EP 18290071**

(43) Date of publication of application:
**05.05.2021 Bulletin 2021/18**

(73) Proprietor: **Koninklijke Philips N.V.**
**5656 AG Eindhoven (NL)**

(72) Inventors:
• **BONNEFOUS, Odile**
**5656 AE Eindhoven (NL)**
• **ZHANG, Bo**
**5656 AE Eindhoven (NL)**
• **DE CRAENE, Mathieu**
**5656 AE Eindhoven (NL)**

(74) Representative: **Philips Intellectual Property &**
**Standards**
**High Tech Campus 52**
**5656 AG Eindhoven (NL)**

(56) References cited:
**US-A1- 2012 022 843     US-A1- 2013 245 441**
**US-A1- 2016 228 190**

**Description**

FIELD OF THE INVENTION

This invention relates to the field of ultrasound imaging, and in particular to the field of color Doppler ultrasound imaging.

BACKGROUND OF THE INVENTION

Conventional color flow Doppler ultrasound imaging allows the user to qualitatively visualize local flow information. With the help of a color bar, one can have a rough notion of velocity of the flow, as well as an approximate flow orientation. This functionality has been widely deployed on today's echography systems.

An important limitation however, is that conventional color Doppler imaging only quantifies the flow along the transmit beams. Full vector flow quantification can be estimated from Doppler measures by imposing a flow-dynamics model, which is able to estimate the orthogonal complement of the velocity vector. A vector flow-imaging mode has also been proposed that allows users to analyze various parameters of cardiovascular flow distribution, such as ventricular vortices. Likewise, information like pressure gradient can be further derived from Navier-Stokes equations.

Despite the availability of the techniques for flow and pressure estimation, there is a lack of simple tools to allow users to integrate valve tracking, tissue tracking and Doppler measurements into an interface with intuitive and limited interactions.

There is therefore a need to provide an imaging method capable of receiving and integrating simple user interactions without the need for significant additional hardware.

Document US 2013/0245441 discloses a method for determining a pressurevolume loop of a subject using ultrasound, wherein both B-mode and flow information are acquired and used to estimate volume over time and pressure over time, respectively.

The document may be considered to disclose a method for generating a transvalvular pressure quantification, the method comprising: acquiring a plurality of color Doppler ultrasound image frames, wherein the image frames comprise a view of a valve and a cavity, presenting an image frame of the plurality of color Doppler ultrasound image frames to a user; receiving a user input from the user indicating a location of the valve; tracking the location of the valve in the plurality of color Doppler ultrasound image frames based on the user input; computing a flow through the valve and in the cavity and the plurality of color Doppler ultrasound image frames; and estimating the transvalvular pressure of the valve based on the flow.

Document US 2012/0041313 discloses an ultrasonic diagnostic device for measuring an absolute pressure inside the heart with respect to heart-beat time phase.

Document US 2013/0197884 discloses a method for computing advanced heart measurements from medical images and data.

Document US 2012/0022843 discloses a method for patient-specific modelling of the whole heart anatomy, dynamics, hemodynamics and fluid structure interaction from 4D medical image data.

The document US2016228190 A1 may be considered to disclose a method for pressure quantification, the method comprising: acquiring a plurality of color Doppler ultrasound image frames, wherein the image frames comprise a view of a valve and a cavity, tracking the location of the valve in the plurality of color Doppler ultrasound image frames; computing a vector flow through the valve and in the cavity based on the tracked location of the valve and the plurality of color Doppler ultrasound image frames; and estimating the pressure in the vicinity of the valve.

SUMMARY OF THE INVENTION

The invention is defined by the claims.

According to examples in accordance with an aspect of the invention, there is provided a method for generating a transvalvular pressure quantification, the method comprising:

acquiring a plurality of color Doppler ultrasound image frames, wherein the image frames comprise a view of a valve and a cavity;
presenting an image frame of the plurality of color Doppler ultrasound image frames to a user;
receiving a user input from the user indicating a location of the valve;
tracking the location of the valve in the plurality of color Doppler ultrasound image frames based on the user input;
computing a vector flow through the valve and in the cavity based on the tracked location of the valve and the plurality of color Doppler ultrasound image frames; and
estimating the transvalvular pressure of the valve based on the vector flow.

This method provides for a simple user interaction to achieve an accurate pressure quantification across a valve.

The user may simply provide a single user input, such as tapping on a screen, to indicate the valve location, in order to obtain a full estimate of the transvalvular pressure across the plurality of color Doppler ultrasound images due to the automatic tracking of the valve.

In an embodiment, the tracking of the location of the valve comprises speckle tracking.

In an arrangement, the estimation of the transvalvular pressure comprises assuming a constant pressure on the valve location.

By assuming constant pressure at the valve location it is possible establish a spatial reference point within the image frame where the pressure from all other points in said image frame may be assessed. In this way, the accuracy of the final estimate is increased.

In an embodiment, the estimation of the transvalvular pressure is based on the Navier Stokes equation.

In some embodiment, the user input further comprises defining a boundary of the cavity.

The entire cavity boundary may then be automatically tracked during the image sequence, in a similar manner to the valve, thereby increasing the accuracy of the final estimate. The cavity may be a cardiac cavity, such as an atrium or ventricle.

In an embodiment, each image frame of the plurality of color Doppler ultrasound image frames is associated with an acquired ECG signal.

This may be used to align the acquired images with a stage in the heartbeat cycle, thereby increasing the amount of information provided to the user regarding the pressure surrounding the valve across the plurality of images.

In an arrangement, the method further comprises:

> determining ejection and filling phases of the valve based on the tracking of the valve; and
> labelling the plurality of color Doppler ultrasound image frames based on the ejection and filling phase determination.

By labelling the images according to the associated behavior of the valve, it is possible to compare the current estimated pressure across the valve with what may be expected.

In a further embodiment, the detection of ejection and filling phases of the valve is further based on the ECG signal.

This increases the accuracy of the comparison between the current estimated pressure across the valve and what may be expected.

In an arrangement, the method further comprises displaying the estimated transvalvular pressure as a color map.

This provides for the quick assessment of the behavior of the valve thereby increasing the usability of the method.

In an arrangement, the view of the valve comprises a mitral valve and an aortic valve.

In an embodiment, the view of the valve comprises a tri-cuspid valve and a pulmonary valve.

According to examples in accordance with an aspect of the invention, there is provided a computer program comprising computer program code means which is adapted, when said computer program is run on a computer, to implement the method described above.

According to examples in accordance with an aspect of the invention, there is provided an ultrasound system adapted to generate a transvalvular pressure quantification, the system comprising:

> an ultrasound probe adapted to acquire ultrasound image data;
> a processor adapted to acquire a plurality of color Doppler ultrasound image frames based on the ultrasound image data, wherein the image frames comprise a view of a valve and a cavity;
> an interactive display unit adapted to:

>> present an image frame of the plurality of color Doppler ultrasound image frames to a user; and
>> receive a user input from the user indicating a location of the valve;

> wherein the processor is further adapted to:

>> track the location of the valve in the plurality of color Doppler ultrasound image frames based on the user input;
>> compute a vector flow through the valve and in the cavity based on the tracked location of the valve and the plurality of color Doppler ultrasound image frames; and
>> estimate the transvalvular pressure of the valve based on the vector flow.

In an embodiment, the interactive display unit comprises a tactile screen adapted to receive a drawing input of the user for defining a boundary of the cavity.

In an arrangement, the system further comprises an ECG sensor.

These and other aspects of the invention will be apparent from and elucidated with reference to the embodiment(s) described hereinafter.

BRIEF DESCRIPTION OF THE DRAWINGS

Examples of the invention will now be described in detail with reference to the accompanying drawings, in which:

Figure 1 shows an ultrasound diagnostic imaging system to explain the general operation;
Figure 2 shows a method of the invention;
Figure 3 shows a color Doppler image frame;
Figure 4 shows a color Doppler image frame after receive a user input; and
Figure 5 shows a color Doppler image frame after a flow vector and transvalvular pressure estimation.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for generating a transvalvular pressure quantification within a cavity. The method includes acquiring a plurality of color Doppler ultrasound image frames, wherein the image frames comprise a view of a valve, one of which is then presented to a user. The user may then provide an input to indicate the location of the valve within the image frame. The location of the valve is then tracked within the remaining image frames based on the user input. A vector flow is estimated based on the color Doppler image frames and the tracked location of the valve, which may be used to estimate the flow across the valve(s) and in the cavity.

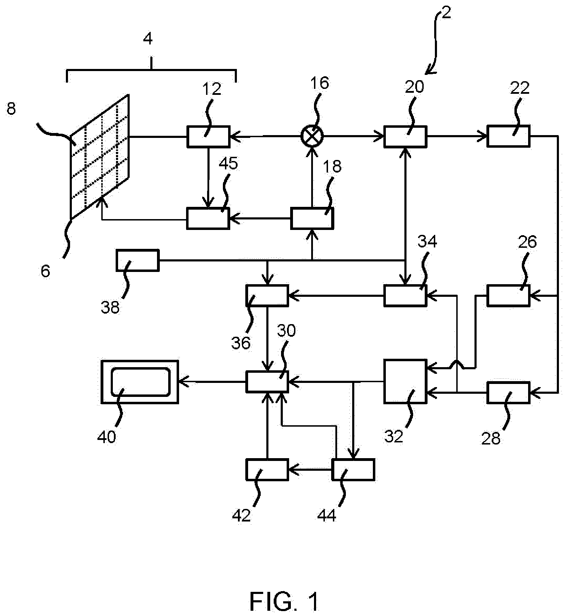

The general operation of an exemplary ultrasound system will first be described, with reference to Figure 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and bandpass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

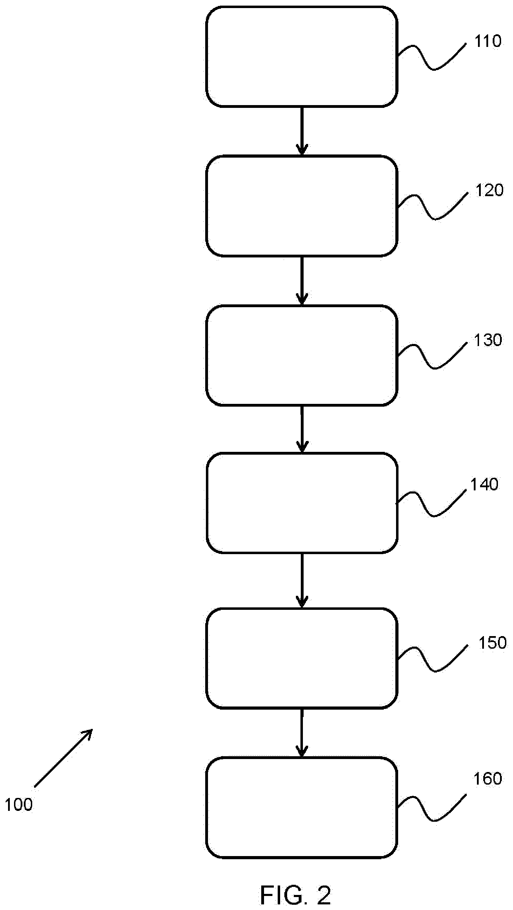

Figure 2 shows a method 100 of the invention.

In step 110, a plurality of color Doppler ultrasound image frames are acquired, wherein the image frames comprise a view of a valve. The valve within the field of view of the image frame may be any valve that the user intends to use as pressure reference.

The plurality of images frames may be acquired on one or multiple cycles and show a single plane in the region of interest, such as the left or right side of the heart.

In examples where the heart is the region of interest the view of the valve may comprise: a mitral valve; an aortic valve; a tri-cuspid valve; or a pulmonary valve. Further, it is possible for more than one valve to be present within the image frames. For example, the view may comprise: a mitral valve and an aortic; or a tri-cuspid valve and a pulmonary valve.

In addition, it may be useful to know exactly where in the heartbeat cycle a particular image is situated, in which case each image frame of the plurality of color Doppler ultrasound image frames may be associated with an acquired ECG signal. This may allow for more in-depth analysis of the state of the valve at a given point of interest in the heartbeat cycle.

In step 120, an image frame of the plurality of color Doppler ultrasound image frames is presented to a user.

This may be the most recently acquired image frame, or any other image frame of the plurality of image frames. The images may be displayed on an interactive display, wherein the user is presented with one of the plurality of color Doppler image frames. The user may view each of the plurality of image frames before proceeding with a desired frame, which may enable the user to select a clearer image if the frame initially presented is unclear.

In step 130, a user input is received from the user indicating a location of the valve.

The user input may comprise tapping the location of the valve on an interactive display surface, such as a touch screen, or it may comprise selecting a location on the image using a digital cursor by way of a mouse, or any suitable input device.

In addition to the locations of the valve in the image frame, the user may provide an input in order to define a cardiac cavity boundary, such as an atrium or a ventricle. In this case, the user may simply select a cavity in order to trigger an automatic segmentation of the indicated cavity. Alternatively, the user may be provided with a tactile screen displaying the image frame in question, wherein the user input comprises the user drawing the outline of the cardiac cavity by hand. Further, it is possible for the segmentation of the cardiac cavity to be performed automatically, without any user interaction.

In step 140, the location of the valve in the plurality of color Doppler ultrasound image frames is tracked based on the user input.

In other words, the user's clicks/taps and the indicated cavity boundary may be automatically tracked along the entire sequence of acquired image frames. The tracking of the location of the valve may be performed using speckle tracking.

The automatic tracking of the valve location throughout the plurality of color Doppler image frames allows the motion of the valve to be discerned throughout the heart beat cycle(s) captured at the acquisition stage. This motion may be married with the associated ECG signal, if present, in order to discern the behavior of the valve, such as filling or ejection, during a particular stage of the heartbeat cycle. This may be used to more easily compare the behavior of the subject's valve to an expected behavior.

The tracking of the valve location may be further used to determine ejection and filling phases of the valve, which may then be used to label the plurality of color Doppler ultrasound image frames. This may be used in further analysis of the plurality of image frames. For example, if the plurality of image frames are labeled with a disproportionate number of either filling or ejection phase labels, the user may discern that there is a problem with the functioning of the valve. This may be further enhances by the inclusion of the associated ECG signal with a given image frame.

In step 150, a vector flow through the valve and across the cavity is computed based on the tracked location of the valve and the plurality of color Doppler ultrasound image frames.

The vector flow estimation may be solved through an optimization framework. To solve the flow vector field $u$, the following framework is minimized:

$$\underset{\nabla \cdot u=0,\ u(B)=v_0}{\arg\min} \quad J(u) = \|M[\langle s, u \rangle - m]\|^2 + \lambda_S \|\nabla u\|^2$$

where: s is the ultrasonic beam orientation along which the color Doppler image frame measures m are acquired; $M$ denotes a mask which defines the sparse area of observed measurement within the color Doppler image frame; $B$ is the flow boundary (tissue) given by the segmentation, on which the boundary velocity $v$ is estimated; and $\lambda_S$ represents the weight of the smoothing term $\|\nabla u\|^2$.

The term $\|M[\langle s, u \rangle - m]\|^2$ restricts the flow solution of the vector field $u$ to be compatible with the Doppler measurements, m. For any pixel, $x$, on the color Doppler image frame, $u(x)$ is the flow vector at said pixel to be solved. The projection of the flow vector $u(x)$ on the Doppler beam orientation vector $s(x)$ at the same pixel is described by their inner product $\langle s(x), u(x) \rangle$. This product is calculated to be as close as possible to the true Doppler measurement at a given pixel. The term $\langle s, u \rangle - m$ is referred to as a difference image and represents the difference between the Doppler measurements, $m$, and the inner product of the flow vector $u(x)$ and the Doppler beam orientation vector $s(x)$.

$M$ is a pixel-wise weighting mask that describes the confidence of each Doppler measurement at each location on the color Doppler image frame. It is applied on the difference image, $\langle s, u \rangle - m$. In the simplest case, at pixel $x$, $M(x)$ is either 0 or 1. As not all pixels have Doppler velocity measurements, only pixels with available measurements will have $M(x) = 1$. The others will have a value of 0 (as no measurement exists). More generally, $M(x)$ ranges between 0 and 1, to reflect prior confidence levels on the Doppler measure at pixel x (for example, as a function of the noise level). For this reason, this first term, $\|M[\langle s, u \rangle - m]\|^2$, should be minimized.

The term $\|\nabla u\|^2$ is for regularization, or smoothing, such that the numerical solution of the equation may be more stable. $\lambda_S$ is a trade-off scalar between the compatibility term (described below) and the smoothing term.

Constraint $u(B) = v_0$, also referred to as the compatibility term, requires the flow solution to be compatible with the boundary condition. The boundary pixel set is specified by B. $v_0$ is the boundary velocity that may be derived as described above, for example using speckle-tracking.

Constraint $\nabla \cdot u = 0$ reflects the restriction that the flow solution should be divergence free due to mass conservation.

The equation is solved using an Augmented Lagrangian method; however, other standard numerical methods for convex minimization, such as projected gradient descent, may also be applied.

In other words, the vector flow is solved in the whole field of view such that: the vector flow is compatible with the observed color Doppler measurements within the mask; the mass preservation of the fluid within the field of view is respected through a zero divergence constraint ($\nabla \cdot u = 0$); and the solution is compatible with the estimated the boundary velocity ($u(B) = v_0$). The boundary velocity may be estimated based on the automatic tracking of the user defined cardiac cavity.

In addition to the boundary velocity, the automatic tracking of the user defined cavity may be used as a constraint for determining a direction of the vector flow at a given point in the image. For example, a cavity may not deform evenly, in which case the automatic tracking of the true cavity boundary may increase the accuracy of the generated vector flow over a model that employs a consistent contraction/expansion motion in the cavity walls.

Further, additional constraints may be taken into account in the computation of the vector flow. For example, the tracked location of the valve may act as a further constraint for dictating the direction of the vector flow at a given point in the heart cycle. For example, the automatic tracking of the valve may be used to identify the crossing point between two cardiac cavities, which may be used to constrain the vector flow to only crossing the cavity boundary at the tracked location of the valve. The tracked location of the valve may be used in conjunction with the boundary velocity to build an accurate picture of the vector flow within the image.

It should be noted that the individual flaps of a cardiac valve may be tracked in an image of sufficiently high spatial-temporal resolution, which may be used for improving the boundary definition and hence the vector flow estimation.

The valve-location tracking may be performed using a sparse demons method, such as the method described in O. Somphone, M. D. Craene, R. Ardon, B. Mory, P. Allain, H. Gao, J. Dhooge, S. Marchesseau, M. Sermesant, and E. Saloux, "Fast myocardial motion and strain estimation in 3D cardiac ultrasound with sparse demons," in ISBI, IEEE, Ed., 2013, pp. 1170-1173. However, any suitable tracking method may be used, such as an optical flow method.

In step 160, the transvalvular pressure of the valve is estimated based on the vector flow.

The transvalvular pressure may be estimated by applying the Navier Stokes equation to estimate the relative pressure by assuming constant pressure at the valve location as annotated by the user and automatically tracked by speckle tracking.

As fluid passes from one side of the valve to the other, the relative pressure will change from positive to negative;

however, in the space near the valve, the fluid is likely to experience significant turbulence. This may complicate the pressure estimate as small pockets of negative pressure may occur in the positive pressure side and vice versa. By assuming a constant pressure at the location of the valve, the point at which the pressure changes from positive to negative is provided with certainty thereby simplifying the transvalvular pressure estimate.

In order to quantify the transvalvular pressure, a spatial reference is needed from which the pressure gradient from the flow estimate may be computed using the Navier-Stokes equation. Using the valve location as indicated by the user as the spatial reference is a natural choice. In this way, the relative pressure quantified across the whole image frame is always relative to the valve, regardless of its position.

Because the transvalvular pressure quantification is not an absolute pressure but a relative one, it is not required that a zero value be initially assumed on the valve. It is possible to assume any constant value. Once the relative pressure is estimated, that constant may then be subtracted from the image frame as a whole.

The Navier Stokes equation may be written as:

$$\frac{\delta \boldsymbol{u}}{\delta t} + (\boldsymbol{u} \cdot \nabla)\boldsymbol{u} - v\nabla^2 \boldsymbol{u} = -\nabla h$$

where: **u** is the estimated vector flow; v is the kinematic velocity; and h is the hydraulic head, which is a combination of the internal and external pressure sources assuming a conservative field.

The estimated transvalvular pressure may be displayed as a color map for all points with flow information in the image and the maximum relative transvalvular pressure can be plotted.

The quantification of relative transvalvular pressure may be displayed either as a color map at a given time frame across the cardiac cavities or at a single point over time (pressure plots across different valves for different phases of the heart cycle including filling and ejection) that are automatically detected.

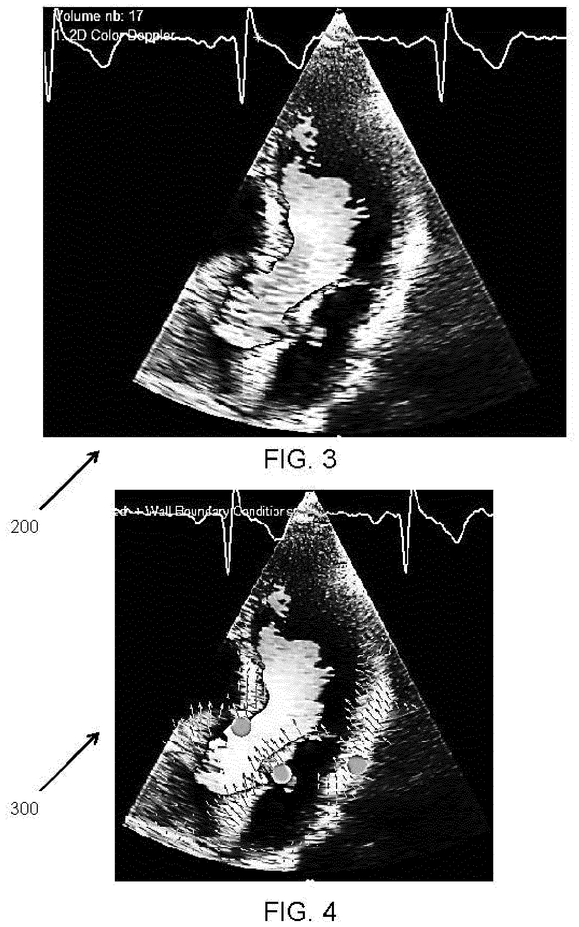

An illustration is given in Figures 3 to 6. Figure 3 shows a conventional color Doppler frame 200 with an associated ECG signal at the top of the image. In this example, this image is presented to a user to indicate the valve locations.

Figure 4 shows a color Doppler image after receiving a user input 300, wherein the locations of the valves, as defined by the user inputs, are represented by three grey circles. As described above, the user only needs to provide input on a single image frame, and these locations are automatically tracked for the following frames.

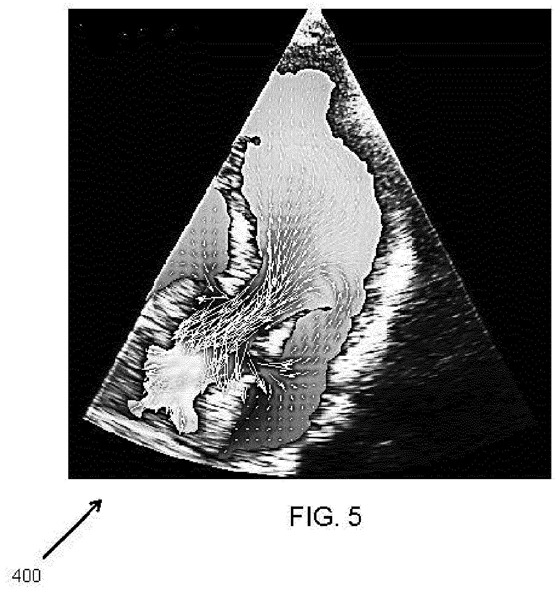

Figure 5 shows a color Doppler image frame including the estimated vector flow and the relative pressure field 400, which may be displayed to a user following the estimation of the transvalvular pressure.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1.  A method (100) for generating a transvalvular pressure quantification, the method comprising:

    acquiring (110) a plurality of color Doppler ultrasound image frames, wherein the image frames comprise a view of a valve and a cavity;
    presenting (120) an image frame of the plurality of color Doppler ultrasound image frames to a user;
    receiving a user input (130) from the user indicating a location of the valve;
    tracking the location of the valve (140) in the plurality of color Doppler ultrasound image frames based on the user input;
    computing a vector flow (150) through the valve and in the cavity based on the tracked location of the valve and the plurality of color Doppler ultrasound image frames; and
    estimating the transvalvular pressure (160) of the valve based on the vector flow.

2.  A method as claimed in claim 1, wherein the tracking of the location of the valve (140) comprises speckle tracking.

3.  A method as claimed in any of claims 1 to 2, wherein the estimation of the transvalvular pressure (160) comprises assuming a constant pressure on the valve location.

4. A method as claimed in any of claims 1 to 3, wherein the estimation of the transvalvular pressure (160) is based on the Navier Stokes equation.

5. A method as claimed in any of claims 1 to 4, wherein the user input further comprises defining a boundary of the cavity.

6. A method as claimed in any of claims 1 to 5, wherein each image frame of the plurality of color Doppler ultrasound image frames is associated with an acquired ECG signal.

7. A method as claimed in any of claims 1 to 6, wherein the method further comprises: determining ejection and filling phases of the valve based on the tracking of the valve; and labelling the plurality of color Doppler ultrasound image frames based on the ejection and filling phase determination.

8. A method as claimed in claims 6 and 7, wherein the detection of ejection and filling phases of the valve is further based on the ECG signal.

9. A method as claimed in any of claims 1 to 8, wherein the method further comprises displaying the estimated transvalvular pressure as a color map.

10. A method as claimed in any of claims 1 to 9, wherein the view of the valve comprises a mitral valve and an aortic valve.

11. A method as claimed in any of claims 1 to 10, wherein the view of the valve comprises a tri-cuspid valve and a pulmonary valve.

12. A computer program comprising computer program code means which is adapted, when said computer program is run on a computer, to implement the method of any of claims 1 to 11.

13. An ultrasound system (2) adapted to generate a transvalvular pressure quantification, the system comprising:

    an ultrasound probe (4) adapted to acquire ultrasound image data;
    a processor adapted to acquire a plurality of color Doppler ultrasound image frames based on the ultrasound image data, wherein the image frames comprise a view of a valve and a cavity;
    an interactive display unit adapted to:

        present an image frame of the plurality of color Doppler ultrasound image frames to a user; and
        receive a user input from the user indicating a location of the valve;

    wherein the processor is further adapted to:

        track the location of the valve in the plurality of color Doppler ultrasound image frames based on the user input;
        compute a vector flow through the valve and in the cavity based on the tracked location of the valve and the plurality of color Doppler ultrasound image frames; and
        estimate the transvalvular pressure of the valve based on the vector flow.

14. A system as claimed in claim 13, wherein the interactive display unit comprises a tactile screen adapted to receive a drawing input of the user for defining a boundary of the cavity.

15. A system as claimed in any of claims 13 to 14, wherein the system further comprises an ECG sensor.

**Patentansprüche**

1. Verfahren (100) zur Erzeugung einer transvalvulären Druckquantifizierung, wobei das Verfahren Folgendes umfasst:

    Erfassen (110) einer Vielzahl von Farbdoppler-Ultraschallbildrahmen, wobei die Bilder eine Ansicht eines Ventils und eines Hohlraums umfassen;
    Präsentieren (120) eines Bildes aus mehreren Farbdoppler-Ultraschallbildrahmen einen Benutzer;
    Empfangen einer Benutzereingabe (130) vom Benutzer, die einen Ort des Ventils angibt;

Verfolgung der Position des Ventils (140) in der Vielzahl von Farbdoppler-Ultraschallbildrahmen auf der Grundlage der Benutzereingabe;
Berechnen eines Vektorflusses (150) durch das Ventil und in der Kavität basierend auf der verfolgten Position des Ventils und mehreren Farbdoppler-Ultraschallbildrahmen; und
Schätzung des transvalvulären Drucks (160) des Ventils auf der Grundlage der Vektorströmung.

2. Verfahren nach Anspruch 1, wobei die Verfolgung der Position des Ventils (140) die Speckle-Verfolgung umfasst.

3. Verfahren nach einem der Ansprüche 1 bis 2, wobei die Schätzung des transvalvulären Drucks (160) die Annahme eines konstanten Drucks an der Ventilstelle umfasst.

4. Verfahren nach einem der Ansprüche 1 bis 3, wobei die Schätzung des transvalvulären Drucks (160) auf der Navier-Stokes-Gleichung basiert.

5. Verfahren nach einem der Ansprüche 1 bis 4, wobei die Benutzereingabe ferner das Definieren einer Begrenzung des Hohlraums umfasst.

6. Verfahren nach einem der Ansprüche 1 bis 5, wobei jedes Bild der Vielzahl von Farbdoppler-Ultraschallbildrahmen einem erfassten EKG-Signal zugeordnet ist.

7. Verfahren nach einem der Ansprüche 1 bis 6, wobei das Verfahren ferner:

   das Bestimmen von Ausstoß- und Füllphasen des Ventils auf der Grundlage der Verfolgung des Ventils umfasst; und
   Kennzeichnung der mehreren Farbdoppler-Ultraschallbildrahmen auf der Grundlage der Ausstoß- und Füllungsphasenbestimmung.

8. Verfahren nach einem der Ansprüche 6 und 7, wobei die Erkennung der Auswurf- und Füllphasen der Klappe ferner auf dem EKG-Signal basiert.

9. Verfahren nach einem der Ansprüche 1 bis 8, wobei das Verfahren ferner das Anzeigen des geschätzten transvalvulären Drucks als Farbkarte umfasst.

10. Verfahren nach einem der Ansprüche 1 bis 9, wobei die Ansicht des Ventils ein Mitralventil und ein Aortenventil umfasst.

11. Verfahren nach einem der Ansprüche 1 bis 10, wobei die Ansicht des Ventils ein Trikuspidalventil und ein Pulmonalventil umfasst.

12. Computerprogramm mit einem Computerprogrammcode, der geeignet ist wenn das Computerprogramm auf einem Computer ausgeführt wird, das Verfahren nach einem der Ansprüche 1 bis 11 zu implementieren.

13. Ein Ultraschallsystem (2), das geeignet ist, eine transvalvuläre Druckquantifizierung zu erzeugen, wobei das System Folgendes umfasst:

   eine Ultraschallsonde (4), die zur Erfassung von Ultraschallbilddaten geeignet ist;
   einen Prozessor, der geeignet ist, mehrere Farbdoppler-Ultraschallbildrahmen auf der Grundlage der Ultraschallbilddaten zu erfassen, wobei die Bildrahmen eine Ansicht eines Ventils und eines Hohlraums umfassen;
   eine interaktive Anzeigeeinheit, die dazu geeignet ist:

      einem Benutzer ein Bild aus mehreren Farbdoppler-Ultraschallbildrahmen zu präsentieren; und
      eine Benutzereingabe des Benutzers zu empfangen, die eine Position des Ventils angibt;

   wobei der Prozessor ferner für Folgendes ausgelegt ist:

      Verfolgung der Position des Ventils mehrerer Farbdoppler-Ultraschallbildrahmen basierend auf der Benutzereingabe;
      Berechnen einer Vektorströmung durch das Ventil und in dem Hohlraum auf der Grundlage der verfolgten Position des Ventils und der mehreren Farbdoppler-Ultraschallbildrahmen; und
Schätzung des transvalvulären Drucks des Ventils auf der Grundlage der Vektorströmung.

14. System nach Anspruch 13, wobei die interaktive Anzeigeeinheit einen taktilen Bildschirm aufweist, der geeignet ist, eine Zeicheneingabe des Benutzers zur Definition einer Begrenzung des Hohlraums zu definieren.

15. Ein System nach einem der Ansprüche 13 bis 14, wobei das System ferner einen EKG-Sensor umfasst.

**Revendications**

1. Procédé (100) pour générer une quantification de la pression transvalvulaire, le procédé comprenant:

   l'acquisition (110) d'une pluralité de trames d'images ultrasonores Doppler couleur, dans laquelle, où les trames d'images comprennent une vue d'une valve et d'une cavité;
   présenter (120) à un utilisateur une trame d'image de la pluralité de trames d'images ultrasonores Doppler couleur;
   recevoir une entrée utilisateur (130) de l'utilisateur indiquant un emplacement de la vanne;
   le suivi de l'emplacement de la valve (140) dans la pluralité de trames d'images ultrasonores Doppler couleur sur la base de l'entrée utilisateur;
   le calcul d'un flux vectoriel (150) à travers la valve et dans la cavité sur la base de l'emplacement suivi de la valve et la pluralité de trames d'images ultrasonores Doppler couleur; et
   l'estimation de la pression transvalvulaire (160) de la valve sur la base du flux vectoriel.

2. Procédé selon la revendication 1, dans lequel le suivi de l'emplacement de la valve (140) comprend un suivi de granularité.

3. Procédé selon l'une quelconque des revendications 1 à 2, dans lequel l'estimation de la pression transvalvulaire (160) comprend l'hypothèse d'une pression constante sur l'emplacement de la valve.

4. Procédé selon l'une quelconque des revendications 1 à 3, dans lequel l'estimation de la pression transvalvulaire (160) est basée sur les équations de Navier Stokes.

5. Procédé selon l'une quelconque des revendications 1 à 4, dans lequel l'entrée utilisateur comprend en outre la définition d'une limite de la cavité.

6. Procédé tel que revendiqué dans l'une quelconque des revendications 1 à 5, dans lequel chaque trame d'image de la pluralité de trames d'images ultrasonores Doppler couleur est associée à un signal ECG acquis.

7. Procédé selon l'une quelconque des revendications 1 à 6, dans lequel le procédé comprend en outre comprend: la détermination des phases d'éjection et de remplissage de la valve sur la base du suivi de la valve; et l'étiquetage de la pluralité de trames d'images ultrasonores Doppler couleur sur la base de la détermination des phases d'éjection et de remplissage.

8. Procédé selon les revendications 6 et 7, dans lequel la détection des phases d'éjection et de remplissage de la valve est en outre basée sur le signal ECG.

9. Procédé selon l'une quelconque des revendications 1 à 8, dans lequel le procédé comprend en outre l'affichage de la pression transvalvulaire estimée sous la forme d'une carte de couleurs.

10. Procédé selon l'une quelconque des revendications 1 à 9, dans lequel la vue de la valve comprend une valve mitrale et une valve aortique.

11. Procédé selon l'une quelconque des revendications 1 à 10, dans lequel la vue de la valve comprend une valve tricuspide et une valve pulmonaire.

12. Programme d'ordinateur comprenant un moyen de code de programme d'ordinateur qui est adapté, lorsque ledit programme informatique est exécuté sur un ordinateur, pour mettre en œuvre le procédé de l'une quelconque des revendications 1 à 11.

13. Système à ultrasons (2) adapté pour générer une quantification de la pression transvalvulaire, le système comprenant:

une sonde à ultrasons (4) adaptée pour acquérir des données d'images ultrasonores;
un processeur adapté pour acquérir une pluralité de trames d'images ultrasonores Doppler couleur sur la base des données d'images ultrasonores, dans lequel les trames d'images comprennent une vue d'une valve et d'une cavité;
une unité d'affichage interactive adaptée pour:

présenter à un utilisateur une trame d'image de la pluralité de trames d'images ultrasonores Doppler couleur; et
recevoir une entrée utilisateur de l'utilisateur indiquant un emplacement de la valve;

où le processeur est en outre adapté pour:

suivre l'emplacement de la valve dans la pluralité de trames d'images ultrasonores Doppler couleur sur la base de l'entrée utilisateur;
calculer un flux vectoriel à travers la valve et dans la cavité sur la base de l'emplacement suivi de la valve et de la pluralité de trames d'images ultrasonores Doppler couleur; et
estimer la pression transvalvulaire de la valve sur la base du flux vectoriel.

14. Système selon la revendication 13, dans lequel l'unité d'affichage interactive comprend un écran tactile adapté pour recevoir une entrée de dessin de l'utilisateur pour définir une limite de la cavité.

15. Système selon l'une quelconque des revendications 13 à 14, dans lequel le système comprend en outre un capteur ECG.

FIG. 1

FIG. 2

FIG. 3

FIG. 4

FIG. 5

400

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130245441 A
- US 20120041313 A
- US 20130197884 A
- US 20120022843 A
- US 2016228190 A1
- US 5997479 A, Savord
- US 6013032 A, Savord
- US 6623432 B, Powers
- US 6283919 B, Roundhill
- US 6458083 B, Jago
- US 6443896 B, Detmer
- US 6530885 B, Entrekin

**Non-patent literature cited in the description**

- Fast myocardial motion and strain estimation in 3D cardiac ultrasound with sparse demons. **O. SOMPHONE ; M. D. CRAENE ; R. ARDON ; B. MORY ; P. ALLAIN ; H. GAO ; J. DHOOGE ; S. MARCHESSEAU ; M. SERMESANT ; E. SALOUX.** ISBI. IEEE, 2013, 1170-1173